# EUROPEAN PATENT SPECIFICATION

(11) **EP 2 277 442 B1**
(45) Date of publication and mention of the grant of the patent: **24.06.2015**
(21) Application number: 10183624.5
(22) Date of filing: 24.02.2003
(51) Int. Cl.: A61B 5/15

(54) **Fluid collection apparatus having an integrated lancet and reaction area**
Vorrichtung zum Sammeln von Flüssigkeiten mit integrierter Lanzette und Reaktionsbereich
Dispositif de collection de fluides avec lancette et domaine de réaction intégrée

(30) Priority: 05.03.2002 US 361405 P
(43) Date of publication of application: 26.01.2011
(62) Divisional of application: 03003256.9
(73) Proprietor: Bayer HealthCare LLC, Whippany, NJ 07981-0915 (US)
(72) Inventor: Brenneman, Allen J., GOSHEN, INDIANA 46526, IN (US)
(74) Representative: BIP Patents

(56) References cited:
- WO-A1-01/72220
- US-A- 5 801 057
- US-A- 5 855 801

## Description

### FIELD OF THE INVENTION

The present invention relates generally to blood monitoring devices and, more particularly, to a fluid collection apparatus having an integrated lance and reaction area for use in determining one or more analytes in a body fluid.

### BACKGROUND OF THE INVENTION

It is often necessary to quickly obtain a sample of blood and perform an analysis of the blood sample. One example of a need for quickly obtaining a sample of blood is in connection with a blood glucose monitoring system where a user must frequently use the system to monitor the user's blood glucose level.

Those who have irregular blood glucose concentration levels are medically required to self-monitor their blood glucose concentration level. An irregular blood glucose level can be brought on by a variety of reasons including illness, such as diabetes. The purpose of monitoring the blood glucose concentration level is to determine the blood glucose concentration level, and then to take corrective action, based on whether the level is too high or too low, to bring the level back within a normal range. The failure to take corrective action can have serious implications. When blood glucose levels drop too low, a condition known as hypoglycemia, a person can become nervous, shaky, and confused. That person's judgment may become impaired and that person may eventually pass out. A person can also become very ill if their blood glucose level becomes too high, a condition known as hyperglycemia. Both conditions, hypoglycemia and hyperglycemia, are potentially life-threatening emergencies.

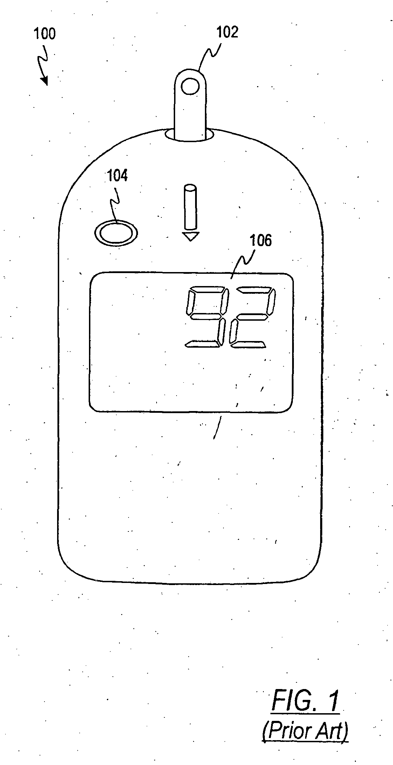

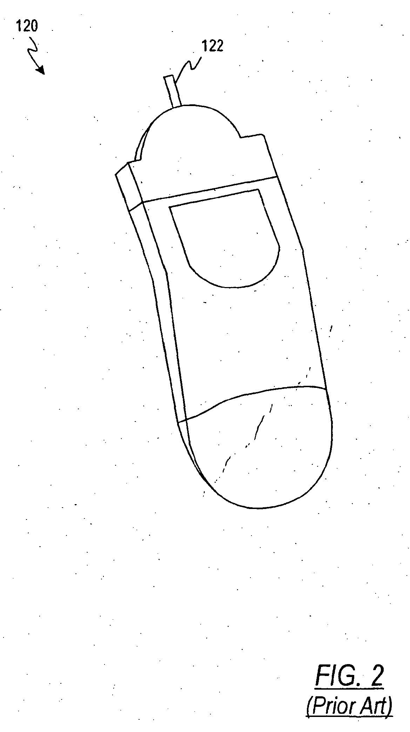

One method of monitoring a person's blood glucose level is with a portable, hand-held blood glucose testing device. A prior art blood glucose testing device 100 is illustrated in FIG. 1. The portable nature of these devices 100 enables the users to conveniently test their blood glucose levels wherever the user may be. The glucose testing device 100 contains a test sensor 102 to harvest the blood for analysis. The device 100 contains a switch 104 to activate the device 100 and a display 106 to display the blood glucose analysis results. In order to check the blood glucose level, a drop of blood is obtained from the body, usually from the fingertip, using a lancing device. A prior art lancing device 120 is illustrated in FIG. 2. The lancing device 120 - contains a needle lance 122 to puncture the skin. Some lancing devices implement a vacuum to facilitate drawing blood. Once the requisite amount of blood is produced on the fingertip, the blood is harvested using the test sensor 102. The test sensor 102, which is inserted into a testing device 100, is brought into contact with the blood drop. The test sensor 102 is filled with blood and creates a color change or an electrical current that is measured by the test device 100, which then determines the to concentration of glucose in the blood. Once the results of the test are displayed on the display 106 of the test device 100, the test sensor 102 is discarded. Each new test requires a new test sensor 102.

One problem associated with many conventional testing systems is that the lance and the sensor are two separate, disposable pieces. Two separate pieces require more assembly work. This is time consuming for the user who must assemble the two disposable pieces prior to use. Also, because there are multiple pieces, there are more pieces for the user to keep track of, reorder, etc. Missing pieces may result in the test not being taken at the appropriate time, or it may result in an additional trip to the store, resulting in further inconvenience to the user.

Another problem associated with current testing devices is the difficulty in harvesting small samples when the sensor is separate from the lance. There is a trend in glucose testing towards smaller and smaller sample volumes. This trend is based on the assumption that there is a corresponding reduction in pain when less sample volume is acquired. As the sample volume is reduced, it becomes more difficult to manually manipulate the sensor in order to harvest the blood. This is especially true for people who may have vision impairments or other disabilities which may make it difficult to manipulate the sensor within a small area.

Another problem associated with obtaining small sample sizes is related to the precision needed to obtain the samples. When small amounts of blood are drawn by the lance, it is important that the entire sample or most of the sample be drawn into the testing device. When larger volumes of blood are drawn, it is less necessary to obtain all of the blood for the sensor. In small volume testing devices, it is advantageous to have the sensor located proximate to the puncture wound to maximize the amount of blood that is drawn into the sensor for testing. In current testing devices, where the sensor has to be manually moved to the puncture wound, it may be difficult to get close enough to the wound to obtain enough of the sample.

Another testing device has been developed for the collection of interstitial fluid (ISF) that utilizes an integrated lance and sensor. ISF is collected by piercing just below the skin before any nerve endings or any capillaries. Collecting ISF is sometimes desirable because there is minimal pain involved since it is above any nerve endings. In this device, the lance and sensor chamber is connected via a capillary channel, all of which are made by etching silicon wafers. This requires numerous steps to form. Furthermore, the lance needle is brittle and requires protection from production to final use. The lance needle and sensor are a single part, but a molded part and a cover are needed to house the integrated sensor for final packaging and use.

Other testing devices have been produced for testing blood that utilize a sensor with a lance perpendicular to the sensor. In this arrangement, the sensor can be positioned to harvest a sample with the lance puncturing the body either through a hole in the sensor or adjacent to the tip of the sensor. When the sample is produced adjacent to the sensor, harvesting of the sample can be automatic and without user judgement. This approach requires precise alignment of both the lancet and the sensor either at the time of manufacture or at the time of use, preferably by the test device, to make it more convenient for the end user.

### SUMMARY OF THE INVENTION

The present invention is a method of manufacturing a fluid collection apparatus that has an integrated lance and reaction area. The method includes providing a sheet of material and then coating the sheet with a photoresist in a pattern on one side of the sheet. The pattern defines a lance and a reaction area. At least one side of the sheet is placed in a solvent and is then corroded in areas not covered by the photoresist. The sheet is removed from the acid after a predetermined time to reveal an integrated lance and reaction area.

The above summary of the present invention is not intended to represent each embodiment or every aspect of the present invention. This is the purpose of the Figures and the detailed description which follow.

### BRIEF DESCRIPTION OF THE DRAWINGS

The foregoing and other advantages of the invention will become apparent upon reading the following detailed description and upon reference to the drawings.
FIG. 1 is atop view of a prior art blood glucose testing device.
FIG. 2 is a perspective view of a prior art lance.
FIG. 3a is a perspective view of a fluid collection apparatus according to one embodiment of the present invention.
FIG. 3b is a side view of the fluid collection apparatus of FIG. 3a.
FIG. 4a is a perspective view of a fluid collection apparatus according to another embodiment of the present invention.
FIG. 4b is a side view of the fluid collection apparatus of FIG. 4a.
FIG. 5 is a view of a first side of a sheet having a mask according to one embodiment of the present invention.
FIG. 6a is a view of a second side of a sheet having a mask according to, one embodiment of the present invention.
FIG. 6b is a view of a second side of a sheet having a mask according to another embodiment of the present invention.'
FIG. 7 is a view of a sheet having a plurality of fluid collection apparatuses according to one embodiment of the present invention.
FIG. 8 is an enlarged view of the circular cut out 8-8 taken from FIG. 7.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the spirit and scope of the invention as defined by the appended claims.

### DESCRIPTION OF ILLUSTRATIVE EMBODIMENTS

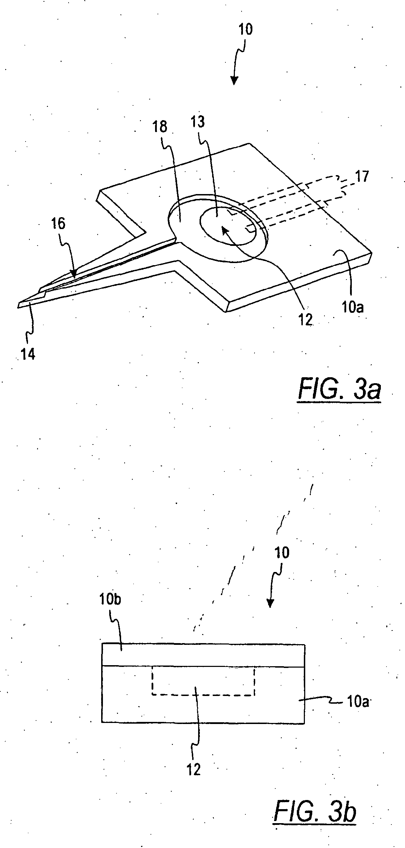

FIG. 3a is a perspective view and FIG. 3b is a side view of a fluid collection apparatus 10 according to one embodiment of the present invention. The fluid collection apparatus 10 is designed to collect a body fluid, for example, blood, so the fluid may be tested for the concentration of a particular analyte, such as glucose. In describing the details of the operation of the fluid collection apparatus 10, the fluid described will be blood pricked from a user's skin and the analyte will be glucose. It is understood that the embodiment may also be used for other fluids and analytes and that these only serve as examples.

The fluid collection apparatus 10 includes a lid 10b and a body 10a (FIG. 3b). The body 10a has a reaction area 12, a lance 14, and a transfer area, such as-a capillary channel 16 (FIG. 3a). According to one embodiment, the reaction area 12, the lance 14, and the capillary channel 16 are all formed of an integrated piece of metal, such as stainless steel. The lance 14 has a nose 15 that is designed to be able to pierce a user's skin (e.g., from a finger tip) to obtain a sample of blood. The nose 15 may be a sharpened point, or it may be two sharpened points, located on opposite sides of the capillary channel 16. The capillary channel 16 couples the, lance 14 to the reaction area 12, such that once the lance 14 pierces the skin of a user, the blood is drawn directly from the point of piercing, up through the capillary channel 16 and into the reaction area 12. The reaction area 12 contains a reagent 13 that is adapted to react with the blood that is drawn into the reaction area 12. A transparent lid (not shown) acts as a cover or top .cover and is located over the top of the reaction area 12. Alternately, the reagent could be deposited on the inside surface of the transparent lid.

According to one embodiment, the fluid collection apparatus 10 can be used in conjunction with a photometric testing device (not shown), which measures a colorimetric reaction. In photometric testing, the reagent 13 used causes a change in color in the reaction area 12. The photometric testing device then measures the amount of color change. Photometric testing is described in more detail in commonly-owned U.S. Patent No. 5,611,999 entitled "Diffuse Reflectance Readhead," which is incorporated herein by reference in its entirety.

In another embodiment of the fluid collection apparatus 10, an electrochemical testing device (not shown) is employed. The reaction area 12 includes a pair of electrodes 17. In electrochemical analysis, the change in current across the electrodes 17 caused by the reaction of the glucose and the reagent 13 creates an oxidation current at the electrodes 17, which is directly proportional to the user's blood glucose concentration. The current can be measured by an electrochemical testing device coupled to a pair of terminals (not shown) corresponding to the electrodes 17. The electrochemical testing device can then communicate to the user the blood glucose concentration. An example of an electrochemical test system is described in detail by commonly-owned U.S. Patent No. 5,723,284 entitled "Control Solution And Method For Testing The Performance Of An Electrochemical Device For Determining The to Concentration Of An Analyte In Blood," which is incorporated herein by reference in its entirety. It is also contemplated that other methods of testing the concentration of glucose in blood may be utilized.

According to the embodiment shown in FIG. 3a, the reaction area 12 has a thickness that is about half the thickness of the fluid collection apparatus 10, which is the thickness of the sheet of material. In these embodiments, the reaction area 12 is bounded on one side by a floor 18 in the fluid collection apparatus 10. These fluid collection apparatuses are also known as being two piece apparatuses. The two piece apparatuses include just the body 10a and the lid 10b (FIG. 3b).

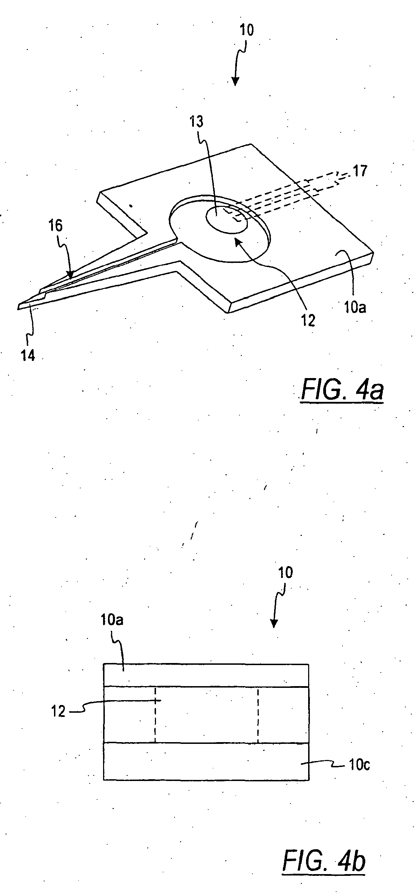

In other embodiments, such as the one shown in FIGS. 4a and 4b, the fluid collection apparatus 10 is a three piece construction, including the body 10a, the lid 10b, and a second cover 10c. In these embodiments, the reaction area 12 has a thickness equal to the thickness of the fluid collection apparatus 10 and/or the sheet of material. The three piece construction is advantageous for an optical transmission design because the light source is on one side and the photodetector is on the other side of the reaction area 12.

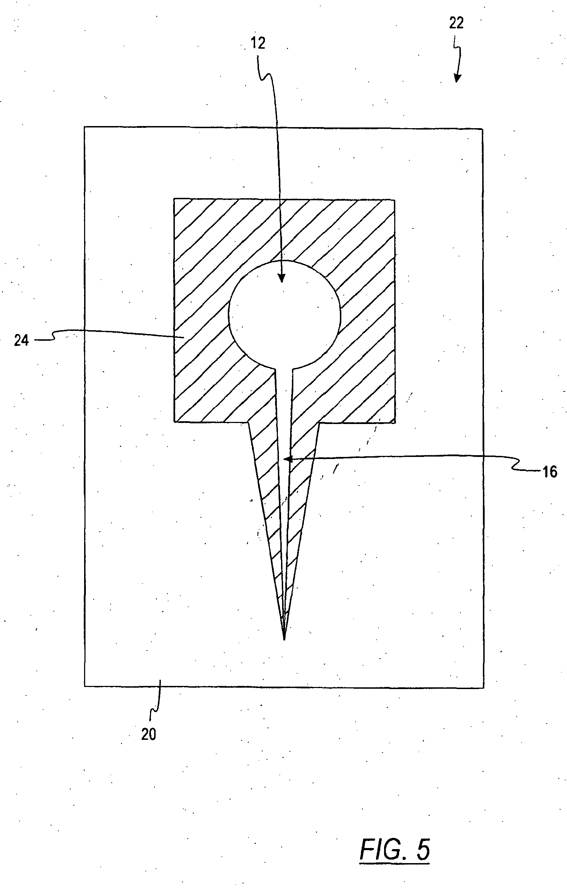

Turning now to FIGS. 5-6b, the process for manufacturing the integrated fluid collection apparatus 10 will be described. As shown in. FIG. 5, a first side 20 of a sheet of material 22 is coated (or masked) in a particular pattern 24 with a photoresist. The pattern 24 is in the shape of the fluid collection apparatus 10. A coating shown by the diagonal lines is formed around the reaction area 12, thus defining the reaction area 12. Similarly, the coating also does not cover, the capillary channel 16 but, instead, defines the channel 16.

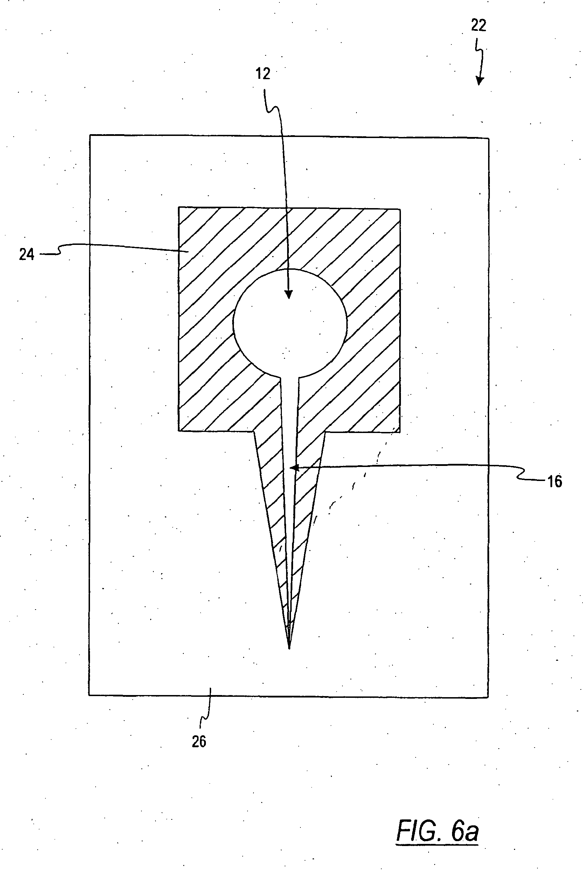

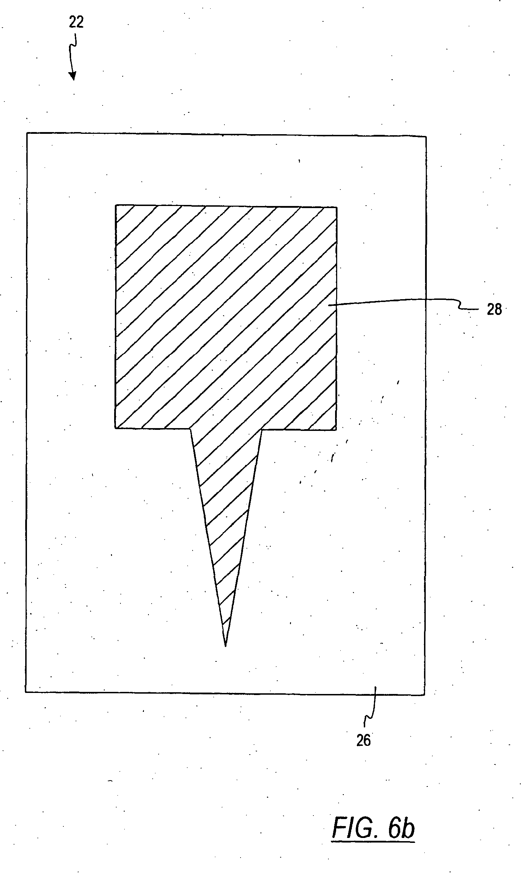

Turning now to FIGS. 6a and 6b, a second side 26 of the sheet 22 is coated with a photoresist. FIG. 6a is the manufacturing of the three piece apparatus, or the apparatus shown in FIG. 4a. In FIG. 6a, the coating on the second side 26 is in the pattern 24 of the first side 20. The reaction area 12 and the capillary channel 16 remain unmasked. The capillary channel could also be masked on the second side, but is not shown. In FIG. 6b, the photoresist is spread in a pattern 28 that extends over the whole shape of the fluid collection apparatus 10. In this embodiment, the reaction area 12 and the capillary channel 16 are coated. This pattern creates the two piece apparatus shown in FIG. 3a.

Once both sides of the sheet 22 have been appropriately coated (for being either a two piece or a three piece apparatus), the sheet 22 is then exposed using lithography. During lithography, the photoresist is hardened by exposing it to ultraviolet light. The sheet 22 is then placed in a solvent, such as an acid. The solvent mills or etches the uncoated portions of the material. The hardened photoresist protects the coated portion of the material from the acid. After a predetermined amount of time (i.e., time sufficient for the solvent to eat through the sheet), the material is removed from the solvent and cleaned.

Thus, the fluid collection apparatus 10 can be Manufactured in only a few steps. Since the lance 14 and the reaction area 12 are one piece, they may be manufactured using this common chemical milling process. By making the lance 14, the capillary channel 16, and the reaction area 12 all one piece, the manufacturing time is reduced, as is the need for extra parts or machines to manufacture the different pieces.

In the embodiment shown in FIG. 6a, the reaction area 12 and the capillary channel 16 are being milled from both sides. Thus, after a predetermined time, the reaction area 12 and the, capillary channel 16 are formed by the acid milling through the entire thickeness of the material. This results in the fluid collection apparatus shown in FIG. 4a.

In the embodiment shown in FIG. 6b, the reaction area 12 and the capillary channel 16 are only left exposed on one side. Thus, the reaction area 12 and the capillary channel 16 will only be milled on one side. In this embodiment, if the sheet of material 22 is kept in the acid for the same amount of time as above, the fluid collection apparatus 10 will have a reaction area 12 and a capillary channel 16 that has half the thickness of the sheet 22. This method results in the fluid collection apparatus shown in FIG. 3a.

In another alternative embodiment of the fluid collection apparatus 10, the first side 20 of the sheet 22 may be milled using a first acid, while the second side 26 is milled using a second, different acid, having a different strength. This way, the acids can be used to create different thicknesses for the reaction area 12 and the capillary channel 16. For example, if a stronger acid is used on the first side 20 than on the second side 26, when the fluid collection apparatus 10 is finished being milled, the stronger acid will have eroded more than half of the sheet 22, thus the thickness of the reaction area 12 and the capillary channel 16 will be greater than half the thickness of the sheet 22. Conversely, if the weaker acid is used on the first side 20, the thicknesses of the reaction area 12 and the capillary channel 16 will be less than half the thickness of the sheet 22.

In the embodiments described above, the fluid collection apparatus 10 typically has a width ranging from about 0.060 to about' 0.090 inches and a length ranging from about 0.120 to about 0.180 inches. The reaction area 12 is shown as generally circular and has a radius ranging from about 0.010 to about 0.030 inches, however, the shape can be oval, diamond, or of a shape to optimize the fluid flow into the reaction chamber. The capillary channel 16 has a width ranging from about 0.001 to about 0.005 inches. The fluid collection apparatus 10 is preferably made of metal, such as stainless steel.

Once the fluid collection apparatus 10 is created, the lid 10b is attached to one side of the fluid collection apparatus. The lid 10b may include the electrochemical electrodes 17 if electrochemical testing is taking place. Alternatively, the lid 10b may be a clear plastic window if optical testing is taking place. In the embodiments where the reaction area 12 and the collection capillary 16 have the same thickness as the material, the second cover 10c is also attached to a side of the fluid• collection apparatus 10.

Now, the operation of the fluid collection apparatus 10 will be described. A user will pierce their skin (e.g., a finger tip) using the lance 14 located on the end of the fluid collection apparatus 10. As blood exits the laceration, the blood is drawn up into the capillary channel 16 ,through capillary action, and into the reaction area 12, where it mixes with the reagent 13, creating a measurable reaction as described .above. After collecting the sample, the fluid collection apparatus 10 is used with a test device (not shown) to measure the reaction. The testing device may be a colorimetric spectrophotometer or current measuring for the electrochemical sensor as described above.

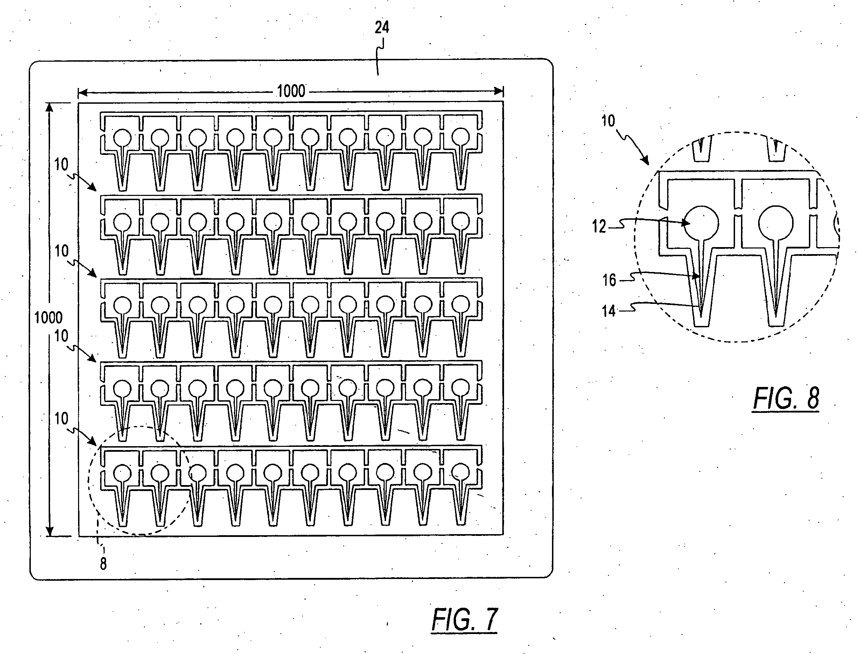

Turning now to FIG. 7, a sheet of material 28 with a plurality of fluid collection apparatuses 10 is depicted. FIG. 8 is an enlarged view of a portion of the sheet 28. In some embodiments, a plurality of fluid collection apparatuses 10 may be to formed on each sheet 28 as shown in FIG. 7. The number of fluid collection apparatuses 10 on each sheet 28 may be modified to suit individual needs. By manufacturing numerous apparatuses 10 on one sheet, many apparatuses 10 can be dipped in the acid at the same time; which enables quick manufacturing of the fluid collection apparatus 10. It is advantageous to be able to mass produce the apparatuses is since that decreases the time and cost of production. Also, there is less sheet of material that is wasted or that needs to be milled by the etchant, which also decreases the manufacturing cost since there is less excess material.

In other embodiments, the fluid collection apparatuses 10 are formed on a continuous web of material. The webs may be manufactured in rolls and continuously fed through the manufacturing machine. Utilizing a continuous web of material also allows for continuous manufacturing of the fluid collection apparatuses 10, which is advantageous since it decreases production costs.

According to alternative embodiments of the present invention, the fluid collection apparatuses 10 may be manufactured by micromachining or, put another way, cutting the fluid collection apparatuses with machinery instead of using acid. For example, the outer edges of the fluid collection apparatuses may be cut using standard machining or lasers. The capillary channel 16 and the reaction area 14 may be manufactured by diamond cutting. 'The reaction area 14 may also be made by lasers, if the reaction area 14 has a thickness equal to the thickness of the sheet. The points of the lance 14 may also be cut by diamond tools or lasers.

While the present invention has been described with reference to one or more particular embodiments, those skilled in the art will recognize that many changes may be made thereto without departing from the spirit and scope of the present invention. Each of these embodiments and obvious variations thereof is contemplated as falling within the scope of the claimed invention, which is set forth in the following claims.

## Claims

1. A fluid collection apparatus (10), comprising:
a lance (14) including a nose for piercing skin to obtain a sample;
a reaction area (12) containing a reagent (13) adapted to react with an analyte in the sample, the reaction providing a measurement of a concentration of the analyte in the sample; and
a capillary channel (16) coupling the lance (14) to the reaction area (12), the capillary channel (16) drawing the sample from the nose of the lance (14) to the reaction area (12),
the lance (14), the reaction area (12) and the capillary channel (16) are integrally fomed from a single material, **characterized in that**
the nose includes two sharpened points located on opposing sides of the capillary channel (16).

2. The fluid collection apparatus (10) of claim 1, wherein the fluid collection apparatus (10) includes a body (10a) and a lid (10b), the lance (14), the reaction area (12) and the capillary channel (16) being formed from the body (10a), and the lid (10b) covering the reaction area (12).

3. The fluid collection apparatus (10) of claim 1 or 2, wherein the reaction area (12) has the thickness of the capillary channel (16).

4. The fluid collection apparatus (10) of any of the claims 1 to 3, wherein the thickness of the capillary channel (16) is approximately half of the body (10a).

5. The fluid collection apparatus (10) of claim 1, wherein the fluid collection apparatus (10) includes a body (10a), a lid (10b), and a cover (10c), the lance (14), the reaction area (12) and the capillary channel (16) being formed from the body (10a).

6. The fluid collection apparatus (10) of claim 5, wherein the reaction area (12) has the thickness of the capillary channel (16).

7. The fluid collection apparatus of any of the claims 1 to 6, wherein the lance (14), the reaction area (12) and the capillary channel (16) are formed from one sheet of metal.

8. The fluid collection apparatus (10) of any of the claims 2, 5 or 6, wherein the lid (10b) is transparent.

9. The fluid collection apparatus (10) of any of the claims 2, 5 or 6, wherein the lid (10b) includes electrochemical electrodes.

10. The fluid collection apparatus (10) of any of the claims 1 to 9, wherein the capillary channel (16) is milled into the body (10a).

11. The fluid collection apparatus (10) of any of the claims 1 to 10, wherein the reaction area (12) is milled into the first side of the body (10a)

## Patentansprüche

1. Vorrichtung (10) zum Sammeln von Flüssigkeiten, umfassend:
eine Lanzette (14) mit einer Nase zum Einstechen in die Haut zur Entnahme einer Probe;
einen Reaktionsbereich (12), der ein Reagenz (13) zur Reaktion mit einem Analyten in der Probe enthält, wobei die Reaktion eine Messung einer Konzentration des Analyten in der Probe bereitstellt; und
einen Kapillarkanal (16), der die Lanzette (14) mit dem Reaktionsbereich (12) verbindet, wobei der Kapillarkanal (16) die Probe von der Nase der Lanzette (14) zu dem Reaktionsbereich (12) zieht,
wobei die Lanzette (14), der Reaktionsbereich (12) und der Kapillarkanal (16) aus einem einzelnen Material einstückig geformt sind, **dadurch gekennzeichnet, dass** die Nase zwei geschärfte Spitzen auf gegenüberliegenden Seiten des Kapillarkanals (16) aufweist.

2. Vorrichtung (10) zum Sammeln von Flüssigkeiten nach Anspruch 1, worin die Vorrichtung (10) zum Sammeln von Flüssigkeiten einen Körper (10a) und einen Deckel (10b) aufweist, wobei die Lanzette (14), der Reaktionsbereich (12) und der Kapillarkanal (16) von dem Körper (10a) geformt werden und der Deckel (10b) den Reaktionsbereich (12) abdeckt.

3. Vorrichtung (10) zum Sammeln von Flüssigkeiten nach Anspruch 1 oder 2, worin der Reaktionsbereich (12) die Dicke des Kapillarkanals (16) aufweist.

4. Vorrichtung (10) zum Sammeln von Flüssigkeiten nach einem der Ansprüche 1 bis 3, worin die Dicke des Kapillarkanals (16) ungefähr die Hälfte des Körpers (10a) beträgt.

5. Vorrichtung (10) zum Sammeln von Flüssigkeiten nach Anspruch 1, worin die Vorrichtung (10) zum Sammeln von Flüssigkeiten einen Körper (10a), einen Deckel (10b) und eine Abdeckung (10c) aufweist, wobei die Lanzette (14), der Reaktionsbereich (12) und der Kapillarkanal (16) von dem Körper (10a) geformt werden.

6. Vorrichtung (10) zum Sammeln von Flüssigkeiten nach Anspruch 5, worin der Reaktionsbereich (12) die Dicke des Kapillarkanals (16) aufweist.

7. Vorrichtung zum Sammeln von Flüssigkeiten nach einem der Ansprüche 1 bis 6,worin die Lanzette (14), der Reaktionsbereich (12) und der Kapillarkanal (16) aus einem Metallblech geformt sind.

8. Vorrichtung (10) zum Sammeln von Flüssigkeiten nach einem der Ansprüche 2, 5 oder 6, worin der Deckel (10b) transparent ist.

9. Vorrichtung (10) zum Sammeln von Flüssigkeiten nach einem der Ansprüche 2, 5 oder 6, worin der Deckel (10b) elektrochemische Elektroden aufweist.

10. Vorrichtung (10) zum Sammeln von Flüssigkeiten nach einem der Ansprüche 1 bis 9, worin der Kapillarkanal (16) in den Körper (10a) gefräst ist.

11. Vorrichtung (10) zum Sammeln von Flüssigkeiten nach einem der Ansprüche 1 bis 10, worin der Reaktionsbereich (12) in die erste Seite des Körpers (10a) gefräst ist.

## Revendications

1. Appareil de collection de fluides (10), comprenant :
une lancette (14) comportant un nez pour percer la peau pour obtenir un échantillon ;
une zone de réaction (12) contenant un réactif (13) prévu pour réagir avec un analyte dans l'échantillon, la réaction fournissant une mesure d'une concentration de l'analyte dans l'échantillon ; et
un canal capillaire (16) accouplant la lancette (14) à la zone de réaction (12), le canal capillaire (16) aspirant l'échantillon depuis le nez de la lancette (14) jusqu'à la zone de réaction (12),
la lancette (14), la zone de réaction (12) et le canal capillaire (16) étant formés intégralement à partir d'un matériau unique, **caractérisé en ce que** le nez comporte deux pointes acérées situées sur des côtés opposés du canal capillaire (16).

2. Appareil de collection de fluides (10) selon la revendication 1, dans lequel l'appareil de collection de fluides (10) comporte un corps (10a) et un couvercle (10b), la lancette (14), la zone de réaction (12) et le canal capillaire (16) étant formés à partir du corps (10a), et le couvercle (10b) recouvrant la zone de réaction (12).

3. Appareil de collection de fluides (10) selon la revendication 1 ou 2, dans lequel la zone de réaction (12) a l'épaisseur du canal capillaire (16).

4. Appareil de collection de fluides (10) selon l'une quelconque des revendications 1 à 3, dans lequel l'épaisseur du canal capillaire (16) est approximativement la moitié du corps (10a).

5. Appareil de collection de fluides (10) selon la revendication 1, dans lequel l'appareil de collection de fluides (10) comporte un corps (10a), un couvercle (10b), et un recouvrement (10c), la lancette (14), la zone de réaction (12) et le canal capillaire (16) étant formés à partir du corps (10a).

6. Appareil de collection de fluides (10) selon la revendication 5, dans lequel la zone de réaction (12) a l'épaisseur du canal capillaire (16).

7. Appareil de collection de fluides selon l'une quelconque des revendications 1 à 6, dans lequel la lancette (14), la zone de réaction (12) et le canal capillaire (16) sont formés d'une tôle de métal.

8. Appareil de collection de fluides (10) selon l'une quelconque des revendications 2, 5 ou 6, dans lequel le couvercle (10b) est transparent.

9. Appareil de collection de fluides (10) selon l'une quelconque des revendications 2, 5 ou 6, dans lequel le couvercle (10b) comporte des électrodes électrochimiques.

10. Appareil de collection de fluides (10) selon l'une quelconque des revendications 1 à 9, dans lequel le canal capillaire (16) est fraisé dans le corps (10a).

11. Appareil de collection de fluides (10) selon l'une quelconque des revendications 1 à 10, dans lequel la zone de réaction (12) est fraisée dans le premier côté du corps (10a).
